(19) Europäisches Patentamt
European Patent Office
Office européen des brevets

(11) Publication number: **0 008 179**
**A2**

# (12) EUROPEAN PATENT APPLICATION

(21) Application number: **79301501.7**

(22) Date of filing: **27.07.79**

(51) Int. Cl.³: **A 61 K 9/00**, A 61 K 9/14

(30) Priority: **04.08.78 GB 3229978**
**15.03.79 GB 7909255**

(43) Date of publication of application: **20.02.80**
**Bulletin 80/4**

(84) Designated Contracting States: **BE CH DE FR GB IT NL SE**

(71) Applicant: **BEECHAM GROUP LIMITED, Beecham House Great West Road, Brentford, Middlesex (GB)**

(72) Inventor: **Clapham, David, 10 Ontario Gardens, Worthing, Sussex (GB)**
Inventor: **Treagust, Peter Glyn, 27 Greenacres, Angmering Village, Littlehampton, W. Sussex (GB)**

(74) Representative: **Dawson, Hugh Bainforde et al, Beecham Pharmaceuticals Great Burgh Yew Tree Bottom Road, Epsom Surrey KT18 5XQ (GB)**

## (54) Powder, process for its preparation, container holding it, and syrup comprising the powder reconstituted with water.

(57) A powder which may be reconstituted into an orally adminstrable syrup by the addition of water, which powder contains a water soluble salt of a penicillin of the formula (I):

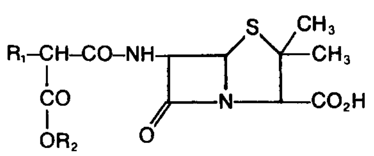

(I)

wherein $R_1$ is phenyl or thienyl, and $R_2$ is phenyl, tolyl or indanyl; in mixture with a solid acidic material; the molar ratio of the penicillin to the acidic material is from 5:1 to 1:5. The powder gives a syrup of improved taste and useful stability.

EP 0 008 179 A2

TITLE MODIFIED
see front page

## POWDERS

The present invention relates to powders which may be reconstituted to form orally adminstrable syrups.

A common method of administering medicaments is in a solution or suspension which has been reconstituted from a powder by the addition of water. For example, certain β-lactam antibiotics such as the orally administrable penicillins are often provided in admixture with excipients such as sugar, colours, preservatives, anti-foams, flavours and the like in the form of a reconstitutable powder. One disadvantage with known formulations of this kind is that they frequently allow the taste of the medicament to be detected and, in the case of an unpleasant tasting medicament, it can cause patient resistance, particularly with children, to taking the medicament.

U.K. Patent No.1511852 describes a powder which may be reconstituted into an orally administrable pharmaceutical composition by the addition of water, which powder comprises (a) fine particles of a water-soluble salt of a carboxylic acid group present in a water-soluble medicament and which fine particles are substantially or wholly coated by a water-soluble coating agent, (b) a water-soluble acidic material and (c) conventional excipients. In these powders, the taste of the medicament is rendered less apparent without seriously impairing its bioavailability.

We have now discovered improved powders for use in this way, which powders are relatively inexpensive and simple to prepare.

Accordingly, the present invention provides a powder which may be reconstituted into an orally administrable syrup by the addition of water, which powder contains a water soluble salt of a penicillin of the formula (I) :

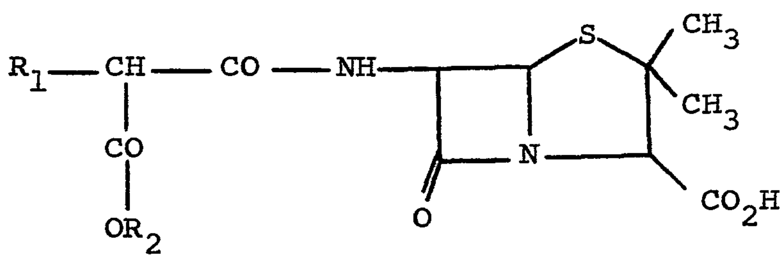

(I)

wherein :

$R_1$ is phenyl or thienyl, and

$R_2$ is a phenyl, tolyl or indanyl group; in mixture with a solid acidic material; the molar ratio of the penicillin to the acidic material is from 5:1 to 1:5.

By "water soluble" is meant being capable of dissolving in water at 20$^{o}$C to an extent which yields at least a 10% solution (weight/volume).

By "acidic material" is meant a pharmaceutically acceptable material which is capable of dissolving in water at 20$^{o}$C to produce a pH of less than 5.

In general, the powder should not contain

significant numbers of penicillin particles of diameter greater than 125 microns in order to avoid an unpalatable feeling of grittiness in the mouth, and, most suitably, at least 90% of these particles should have a diameter less than 125 microns. Preferably this diameter limit is 100 microns.

The water soluble salts of the penicillin of the formula (I) are salts of the free carboxylic acid group present in the penicillin and are usually alkali metal salts such as the sodium or potassium salts of the free carboxylic acid group.

A particularly suitable penicillin of the formula (I) is carfecillin (carfecillin is the phenyl ester of carbenicillin). A preferred penicillin salt for inclusion in the powders of the invention is the sodium salt of carfecillin.

The sodium salts of ticarcillin phenyl and tolyl esters may also be suitably used.

Suitable acidic materials for use in this invention include acids (such as organic acids), and also salts of strong acids with weak bases.

Because of their unobjectional taste and low toxicities, particularly suitable acidic materials for use in this invention include natural non-toxic fruit acids such as citric, tartaric, malic and ascorbic acids; natural non-toxic dibasic acids such as gluconic, maleic and succinic acids; non-toxic tri-basic acids such as aconitic acid; and acid addition salts of natural non-toxic amino-acids such as glycine and glutamine. If desired mixtures of such acidic materials may be used.

Citric, tartaric, malic, maleic and aconitic acids and glycine hydrochloride are examples of particularly suitable acidic materials for use in the powders of this invention.

Preferred acidic materials include a mixture such as :

(a) Citric, tartaric and malic acids; especially a mixture containing two parts of citric acid, four parts of tartaric acid and three parts of malic acid ('parts' being parts by weight);

(b) Malic and maleic acids, and glycine hydrochloride; especially a mixture containing four parts of malic acid, one to two parts of maleic acid and three parts of glycine hydrochloride; and

(c) Aconitic and tartaric acids; expecially a mixture containing equal parts of each acid.

The acidic material should be present in the powder in particles of size in the range 50-500 microns, though due to the solubility of the acidic material this is not an important consideration. Examples of suitable sizes are as for the penicillin particles as hereinbefore described.

The ratio of penicillin to acidic material present expressed on a molar basis is from 5:1 to 1:5, for example 2:1 to 1:5 and more suitably from 2:1 to 1:3. We have found that praticularly good results are obtained when this ratio is 2:1 to 1:1, for example 1.25:1.

From the aforesaid it may be seen that a preferred embodiment of the invention is a powder comprising sodium carfecillin in mixture with a solid acidic material; the molar ratio of the penicillin to the acidic material is from 2:1 to 1:3.

Suitable and preferred examples of acidic materials and molar ratios in this embodiment are as hereinbefore described.

The powders of this invention will normally of course contain conventional excipients found in conventional reconstitutable powders. For example, the powders may contain sugar, flavours, artificial

sweeteners, preservatives, surfactants, anti-foams, and the like in conventional quantities. Such excipients will be well known to those skilled in formulating penicillins or cephalosporins , and will of course be chosen so as to be compatible with the other powder ingredients both in the dry form and on reconstitution.

The powders of the present invention may be presented in multidose form for example in bottles or similar containers, or in single dose form for example in sachets or the like. When reconstituted the powders of the invention have acceptable stability.

Normally the powders of this invention will be in multi-dose bottles such that when they are reconstituted with water the resultant suspension will contain 125 mg or 250 mg of free penicillin per 5 ml of suspension.

Suitably the constitution of the powders of this invention will be such that the pH value of the suspension resulting from the admixture of the powders with water is in the range 1.5 to 4.5.

The powders of this invention may be prepared by mixing methods such as those conventionally used in the preparation of reconstitutable pharmaceutical penicillin or cephalosporin powders.

Suitable methods of mixing include mixing in a 'Y-cone blender'. (Y-cone is a Registered Trade Mark).

The particles of penicillin and acidic material may be prepared by methods of comminution conventionally used in the preparation of pharmaceutical particles, such as by fine milling.

The penicillin and acidic material are preferably comminuted separately. Where the acidic material is a mixture of different acidic materials, the components thereof may also be comminuted either together or separately.

The invention also provides a method of treatment of bacterial infections, which method comprises administering to the sufferer an effective amount of a reconstituted powder of this invention.

The 'effective amount' will be the quantity of powder necessary to give an effective dose of the penicillin included therein.

In an alternative aspect, this invention provides a method of improving the taste of syrups reconstituted from powders containing a water soluble salt of a penicillin of formula (I), which method comprises including in the syrup a solid acidic material in the hereinbefore defined molar ratio.

The following Examples illustrate the invention.

EXAMPLE 1

A reconstitutable powder was prepared from :

| | | | |
|---|---|---|---|
| Sodium carfecillin | | 250 | mg |
| Citric acid | | 25 | mg |
| Tartaric acid | | 50 | mg |
| Malic acid | | 37.5 | mg |
| Flavours | | 32.5 | mg |
| Sodium chloride | | 20 | mg |
| Saccharin sodium | | 10 | mg |
| Sodium lauryl sulphate | | 1.25 | mg |
| Sugar | to | 3.75 | g |

Sodium carfecillin and the three fruit acids were micronised together and then blended with the remaining components.

EXAMPLE 2

The process described in Example 1 was repeated omitting the sodium chloride, saccharin sodium and sodium lauryl sulphate.

EXAMPLES 3 to 10

The process described in Example 1 was repeated, substituting the mixture of tartaric, malic and citric acids used therein by the acidic material shown in the Table below :

Table

| Example No. | Acidic Material | Weight, mg |
|---|---|---|
| 3 | Aconitic acid | 137.5 |
| 4 | Ascorbic acid | 185.0 |
| 5 | Citric acid | 110.0 |
| 6 | Malic acid | 75.0 |
| 7 | Maleic acid | 61.0 |
| 8 | Tartaric acid | 160.0 |
| 9 | Succinic acid | 62.0 |
| 10 | Glycine hydrochloride | 116.0 |

(In these Examples 3 to 10 the penicillin and acid were fine milled rather than micronised as in Example 1).

EXAMPLE 11

The penicillin salt and fruit acids of Example 1 were finely milled together to a majority particle size of approximately 125 µm or less and then blended with the remaining components of Example 1.

EXAMPLE 12

The process of Example 11 was carried out except that the penicillin salt and the fruit acids were finely milled separately.

EXAMPLE 13

A reconstitutable powder was prepared from :

| | |
|---|---|
| Sodium carfecillin | 250.0 mg |
| Aconitic acid | 55.0 mg |
| Tartaric acid | 52.5 mg |

and conventional quantities of flavours, artificial sweeteners and thickeners.

EXAMPLE 14

The powders prepared according to each of the Examples 1 to 13 were made up to 5 ml. with water to form orally administrable pleasant-tasting syrups.

EXAMPLE 15

The syrup prepared from the powder of Example 1 was tested for stability, and was found to be of acceptable stability for at least ten days at $15^{\circ}$ C.

EXAMPLE 16

The following syrup was prepared :

| | | |
|---|---|---|
| Sodium carfecillin | | 250.0 mg |
| Sodium chloride | | 20.0 mg |
| Saccharin Sodium | | 10.0 mg |
| Sodium Lauryl Sulphate | | 1.25mg |
| Thickener | | 10.0 mg |
| Flavours | | 32.5 mg |
| Maleic Acid | | 20.0 mg |
| Malic Acid | | 47.0 mg |
| Glycine Hydrochloride | | 38.0 mg |
| Sugar | to | 3.75 g |
| Water | to | 5.0 ml |

In a blood level study in humans this syrup gave useful bioavailability of carfecillin.

EXAMPLE 17

The following powders (a) to (d) were separately prepared. In each case the drug was fine milled to a majority particle size of 125 μm or less, and then mixed with the other ingredients.

| | | | | |
|---|---|---|---|---|
| (a) | Sodium carfecillin | | 250 | mg |
| | Maleic Acid | | 4 | mg |
| | Malic Acid | | 11.75 | mg |
| | Glycine Hydrochloride | | 9.5 | mg |
| | Flavours | | 100 | mg |
| | Sodium Chloride | | 20 | mg |
| | Saccharin Sodium | | 10 | mg |
| | Surfactant | | 1 | mg |
| | Thickener | | 12 | mg |
| | Sugar | to | 3.75 | g |

| | | | | |
|---|---|---|---|---|
| (b) | Sodium carfecillin | | 250 | mg |
| | Maleic Acid | | 8 | mg |
| | Malic Acid | | 23.5 | mg |
| | Glycine Hydrochloride | | 19 | mg |
| | Flavours | | 100 | mg |
| | Sodium Chloride | | 20 | mg |
| | Saccharin Sodium | | 10 | mg |
| | Surfactant | | 1 | mg |
| | Thickener | | 12 | mg |
| | Sugar | to | 3.75 | g |
| (c) | Sodium carfecillin | | 250 | mg |
| | Maleic Acid | | 3.7 | mg |
| | Malic Acid | | 9.3 | mg |
| | Glycine Hydrochloride | | 7.5 | mg |
| | Sodium Chloride | | 20 | mg |
| | Saccharin Sodium | | 10 | mg |
| | Thickener | | 12 | mg |
| | Flavours | | q.s. | |
| | Surfactant | | q.s. | |
| | Sugar | to | 3.75 | g |
| (d) | Sodium carfecillin | | 250 | mg |
| | Maleic Acid | | 28 | mg |
| | Malic Acid | | 23.5 | mg |
| | Saccharin Sodium | | 14 | mg |
| | Sodium Chloride | | 20 | mg |
| | Surfactant | | 1 | mg |
| | Thickener | | 12 | mg |
| | Flavours | | q.s. | |
| | Sugar | to | 3.75 | g |

EXAMPLE 18

The powders of Example 17 (a) and (b) were prepared again in exactly similar manner except that in each case 500 mg of flavours were used.

EXAMPLE 19

The powder of Example 17 (b) was prepared again in exactly similar manner except that the sodium carfecillin was replaced by 250 mg of the sodium salt of ticarcillin phenyl ester.

EXAMPLE 20

The powder of Example 17 (b) was prepared again in exactly similar manner except that the sodium carfecillin was replaced by 250 mg of the sodium salt of ticarcillin tolyl ester.

EXAMPLE 21

The powders of Examples 17 to 20 were separately filled in unit dose sachets.

EXAMPLE 22

The powders of Examples 17 to 20 were separately made up to 5 ml with water to form orally administrable pleasant tasting syrups.

What we claim is:

1. A powder which may be reconstituted into an orally administrable syrup by the addition of water, which powder contains a water soluble salt of a pencillin of the formula (I):

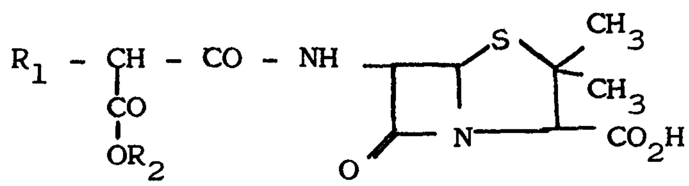

(I)

wherein $R_1$ is phenyl or thienyl, and $R_2$ is phenyl, tolyl or indanyl; in mixture with a solid acidic material; the molar ratio of the penicillin to the acidic material is from 5:1 to 1:5.

2. A powder according to claim 1, wherein the molar ratio is 2:1 to 1:5.

3. A powder according to claim 2, wherein the molar ratio is 2:1 to 1:3.

4. A powder according to any one of the preceding claims, wherein the pencillin is sodium carfecillin.

5. A powder according to any one of the preceding claims, wherein the acidic material is citric, tartaric, malic, maleic or aconitic acid, or glycine hydrochloride.

6. A powder according to any one of the preceding claims, wherein the acidic material is a mixture of acids.

7. A container holding a single or multi-dose of the powder according to any one of the preceding claims.

8. A syrup comprising a powder according to any one of the preceding claims reconstituted with water.

9. A process for the preparation of a powder according to any one of the claims 1 to 6, which process comprises mixing together the powder ingredients.